# EUROPÄISCHE PATENTSCHRIFT

(11) **EP 0 638 298 B1**
(45) Veröffentlichungstag und Bekanntmachung des Hinweises auf die Patenterteilung: **01.07.1998**
(21) Anmeldenummer: 94106518.7
(22) Anmeldetag: 26.04.1994
(51) Int. Cl.: A61C 19/00, A61B 19/00, A47L 15/50, A61L 2/26

(54) **Kassette zur Aufnahme ärztlicher, insbesondere zahnärztlicher, Instrumente**
Cassette for storing medical, especially dental instruments
Cassette pour emmagasiner des instruments médicaux, notamment dentaires

(30) Priorität: 15.07.1993 DE 9310601 U
(43) Veröffentlichungstag der Anmeldung: 15.02.1995
(73) Patentinhaber: Sirona Dental Systems GmbH & Co.KG, 64625 Bensheim (DE)
(72) Erfinder: Hruza, David, Dipl.-Ing., D-88339 Bad Waldsee (DE); Stetter-Alle, Raimund, Dipl.-Ing. (FH), D-89073 Ulm (DE); Trackl, Karl, D-89129 Langenau (DE); Sutter, Ralf, Dipl.-Ing. (FH), D-69469 Weinheim (DE); Beerstecher, Lutz, Dipl.-Ing., D-64625 Bensheim (DE)
(74) Vertreter: Isenbruck, Günter, Dr.

(56) Entgegenhaltungen:
- EP-A- 0 300 945
- CH-A- 657 516
- DE-A- 3 117 264
- DE-A- 3 232 329
- DE-A- 3 443 912
- DE-U- 8 014 994
- DE-U- 9 310 601

## Beschreibung

Die Erfindung bezieht sich auf eine Kassette zur Aufnahme von ärztlichen, insbesondere zahnärztlichen Instrumenten, die zum Zwecke einer hygienischen Behandlung in einer druckdicht verschließbaren Kammer eines Gerätes einlegbar sind und dort einem Behandlungsprozeß unterworfen werden, wobei die Kassette mindestens einen Adapter zur Halterung mindestens eines Instruments aufweist.

Eine solche Kassette ist beispielsweise aus DE-A-3 117 264 bekannt. Die dort vorgestellte Kassette dient hauptsächlich zur Aufbewahrung der Instrumente während und nach einer Sterilisation der Instrumente und ist zweiteilig ausgebildet. In der unteren Kassettenhälfte werden die Instrumente auf in waagerechten Ständern befindlichen Aufnahmeeinrichtungen abgelegt. Die obere Hälfte ist als Deckel ausgebildet Eine zu ähnlichen Zwecken vorgesehene Kassette ist aus EP-A-0 429 960 bekannt.

Beide vorgenannten Kassetten dienen in erster Linie zur Halterung bzw. Aufbewahrung der Instrumente während bzw. nach einer Sterilisation, wobei während der Sterilisationsphase nur die äußeren Flächen der Instrumente durch Heißdampf beaufschlagt werden. Eine darüber hinausgehende Behandlung der Instrumente, insbesondere Reinigung und Pflege derer Innenteile, ist mit diesen Kassetten nicht möglich.

Aus der DE-A1-3 443 912 schließlich ist eine Laborgeschirrspülmaschine zur Aufnahme und Halterung von flexiblen Endoskopen bekannt, bei der die Endoskope in einem Traggestell wendel- oder spiralförmig in mehreren Ebenen übereinanderliegend abgelegt werden können. Die Anschlußteile der Endoskope können an ein Rohrsystem angeschlossen werden, welches mit der Spülmaschine kuppelbare Anschlußstutzen zur Zufuhr von Spülwasser und/oder Trockenluft aufweist. Mit dieser Einrichtung lassen sich die Endoskope innen und außen reinigen.

Der im Anspruch 1 angegebenen Erfindung liegt die Aufgabe zugrunde, eine Kassette der eingangs genannten Gattung anzugeben, die nicht nur alleine zur Halterung der Instrumente, sondern auch zu deren äußeren und inneren Reinigung, Sterilisation und Pflege eingesetzt werden kann.

Die erfindungsgemäß vorgestellte Kassette hat den Vorteil, daß nur den auf den Adapter aufgesetzten Instrumenten Reinigungsmedium zur Behandlung der Innenteile der Instrumente zugeführt wird. Die auf die Adapter aufgesetzten Instrumente lassen sich so innen optimal behandeln, d.h. reinigen, desinfizieren, mit Schmieröl od.dgl. pflegen und auch sterilisieren. Die nach der Patientenbehandlung in die Kassette gegebenen Instrumente können bis zur vollständigen hygienischen Behandlung darin verbleiben. Sie brauchen zwischendurch nicht von einer Bedienperson in die Hand genommen zu werden und, wie dies bisher beim Stand der Technik der Fall ist, zur Pflege in eine spezielle Pflegeeinrichtung und von dort aus wieder in eine separate Sterilisationseinrichtung gegeben zu werden.

Ein Ausführungsbeispiel der Erfindung wird nachfolgend anhand der Zeichnung näher erläutert.

Es zeigen:
Figur 1 eine Ausführungsform der erfindungsgemäßen Vorrichtung in einer schaubildlichen Darstellung,
Figur 2 eine Ausführungsform einer in die Vorrichtung nach Figur 1 einsetzbaren Instrumentenkassette in schaubildlicher Explosionsdarstellung,
Figuren 3 und 4 eine Ausführungsform einer Abdeckung an der Rückseite der in Figur 2 gezeigten Kassette,
Figur 5 einen der in Figur 2 gezeigten Adapter und den rückwärtigen Teil eines dazu passenden Instruments im Längsschnitt,
Figur 6 eine vereinfachte Darstellung der Anordnung einer mit Instrument bestückten Kassette im Gerät.
Figur 7 ein hydraulisch/pneumatisches Schaltbild.
Figur 8 ein Impuls-Zeitdiagramm,
Figur 9 eine Variante zu der in Figur 6 gezeigten Ausführungsform.

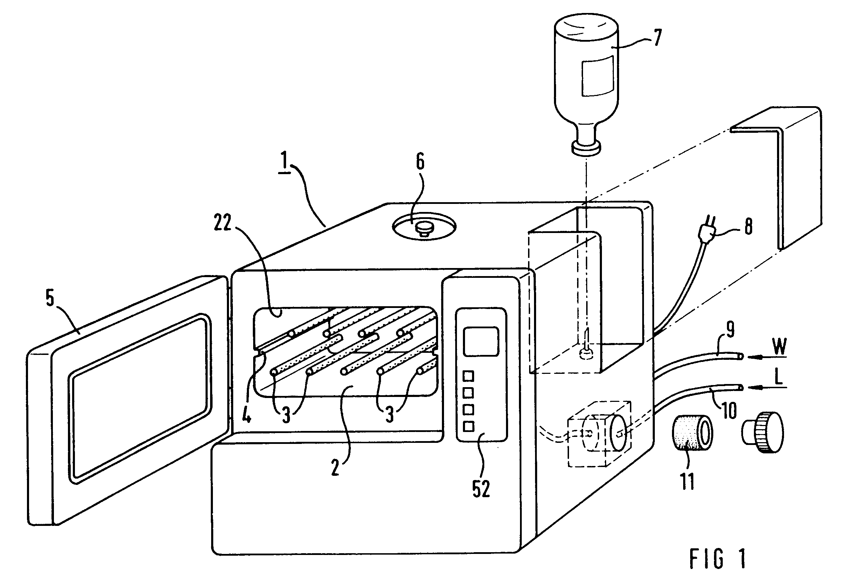

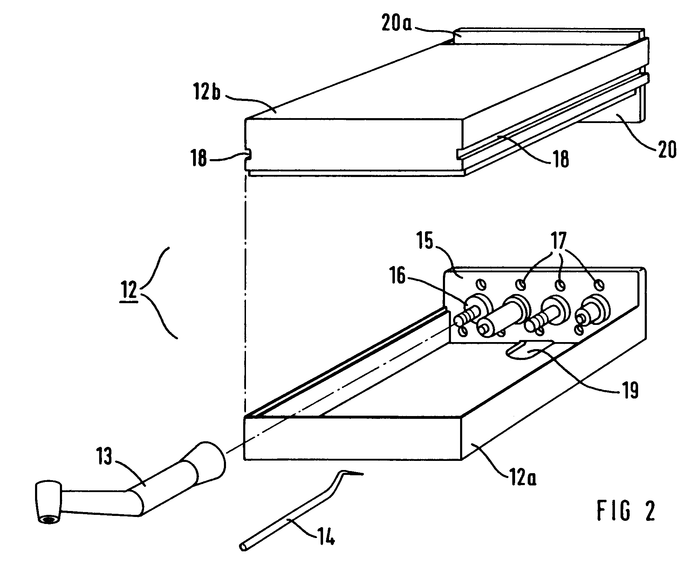

Die Figur 1 zeigt in einer schaubildlichen Darstellung eine Ausführungsform des erfindungsgemäßen Gerätes, welches hier zur hygienischen Aufbereitung diverser zahnmedizinischer Instrumente dient. Das Gerät enthält ein allgemein mit 1 bezeichnetes Gehäuse, welches frontseitig eine Kammer 2 zur Aufnahme einer später noch näher erläuterten Kassette bildet. In die Kammer 2 ragen eine Vielzahl (hier neun) von Düsenarmen 3, mit deren Hilfe, wie ebenfalls später noch näher erläutert, die Instrumente außen besprüht werden können. Beidseitig der Kammer 2 sind Führungsleisten 4 zur Führung der in Figur 2 näher dargestellten Kassette angeordnet. Die Kammer 2 ist in bekannter Weise frontseitig mittels eines Deckels 5 druckdicht verschließbar.

Im rückwärtigen Teil des Gehäuses 1 befindet sich an der Oberseite die Einfüllöffnung 6 eines nicht dargestellten Behälters für demineralisiertes bzw. destilliertes Wasser. Seitlich daneben ist eine abdeckbare Nische zur Aufnahme eines Schmierölvorratsbehälters 7 vogesehen. Dieser Behälter kann in bekannter Weise einen Membranverschluß aufweisen, der beim Aufsetzen auf einen entsprechend ausgebildeten Anschlußzapfen von diesem durchstochen wird, wodurch eine Entnahme des Schmieröls ermöglicht ist. Mit 8, 9 und 10 sind Zuleitungen für elektrische Energie, Druckwasser sowie Druckluft bezeichnet. Mit 11 ist ein leicht entnehmbares und damit austauschbares Sterilfilter bezeichnet, welches im Leitungskanal der Druckluftzuleitung einsetzbar ist. Damit kann sichergestellt werden, daß weitgehend keimfreie Luft für die nachfolgend näher erläuterten Verfahrensschritte zur Verfügung steht.

Die Figur 2 zeigt in einer schaubildlichen Explosionsdarstellung eine in die Kammer 2 einsetzbare Kassette 12 zur Aufnahme mehrerer Instrumente 13 und 14, die hygienisch aufbereitet werden sollen. Das in der Figur mit 13 bezeichnete Instrument ist ein Hand- und Winkelstück, welches im Innern diverse Antriebsteile und Lager sowie Medienkanäle aufweist. Das Instrument 14 dagegen ist ein Handinstrument, welches keine bewegten Teile und auch keine Medienkanäle aufweist.

Die Kassette 12 besteht aus einem Basis- oder Unterteil 12a und einem abnehmbar daran gehalterten Kassettenoberteil 12b. Das Kassettenunterteil 12a ist wannenförmig ausgebildet und enthält an einer erhöhten Rückwand 15 vier verschiedene Adapter zur Aufnahme unterschiedlich gestalteter Instrumente. Im dargestellten Ausführungsbeispiel ist der Adapter 16 auf das aufzunehmende Instrument 13 abgestimmt. Die vier Adapter sind für Instrumente vorgesehen, die bewegte Innenteile und Medienkanäle aufweisen, wie z.B. Hand- und Winkelstücke, Turbinen-, Zahnsteinentfernungs- oder Spritzhandstücke. Instrumente, die keine bewegten Innenteile haben und auch keine Medienkanäle beinhalten, wie beispielsweise das in der Figur mit 14 bezeichnete Handinstrument, werden zur Aufbereitung mittels geeigneter Halterungen am Boden des Kassettenunterteils plaziert.

Die Rückwand 15 des Kassettenunterteils 12a enthält (neun) Bohrungen 17, durch die beim Einsetzen der Kassette 12 in die Kammer 2 die dort vorhandenen (neun) Düsenarme 3 hindurchgreifen können. Die Anordnung der Düsenarme 3 und der mit ihnen korrespondierenden Bohrungen 17 sind, wie aus der Abbildung hervorgeht, jeweils dreieckförmig um die mittig gelegenen Adapter angeordnet.

Das auf das Kassettenunterteil 12a aufsetzbare und mit diesem rastend verbindbare Oberteil 12b enthält seitliche Führungsnuten 18, die mit den bereits erwähnten Führungsleisten (Pos. 4 in Fig. 1) zusammenwirken und eine exakte Zentrierung und Führung der Kassette in der Kammer 2 gewährleisten.

Die Kassette ist an geeigneter Stelle, vorzugsweise bodenseitig und dicht benachbart der Rückwand 15 mit einer oder mehreren Ablauföffnungen 19 für den Ablauf des zugeführten Reinigungs- sowie Kondenswasser versehen.

Vorteilhaftweise ist (sind) diese Ablauföffnung(en) mit einem beim Entnehmen der Kassette selbsttätig verschließenden Teil, z.B. mit einem federbelasteten Schieber oder Deckel, versehen.

Das Kassettenoberteil 12b ist vorteilhafterweise an der Rückseite mit einer Abdeckung versehen sein, welche die Öffnungen 17 und die zu den Adaptern 16 führenden Anschlüsse sowie gegebenenfalls die Ablauföffnung 19 abdeckt bzw. verschließt, wenn die Kassette aus dem Gerät herausgenommen wird.

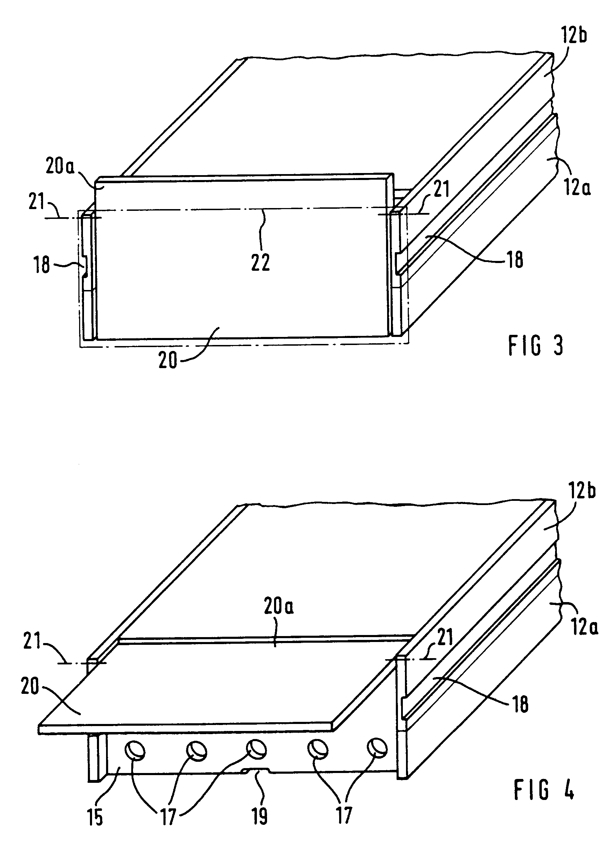

Die Figuren 3 und 4 zeigen eine Ausführungsform einer solchen Abdeckung, wobei die Figuren die Kassette von der Rückseite zeigen. Eine Abdeckplatte 20 ist um ein Lager 21 nach oben aufklappbar. Das Aufklappen geschieht automatisch beim Einschieben der Kassette 12 in die Kammer 2 des Gerätes 1. Hierzu weist die Abdeckplatte 20 einen Überstand 20a auf, der beim Einschieben der Kassette in die Kammer 2 an der mit 22 angedeuteten Oberkante der Kammer 2 anschlägt, wodurch die Klappe nach oben geöffnet wird (Fig. 4). Die sich dahinter befindlichen Öffnungen 17 sowie die aus der Abbildung nicht ersichtlichen Anschlüsse zu den Adaptern 16 werden sodann freigegeben. Beim Herausnehmen der Kassette klappt die Abdeckplatte 20 automatisch wieder nach unten und verschließt so die Öffnungen, so daß die in der Kassette befindlichen, hygienisch aufbereiteten Instrumente gegen Eindringen von Keimen zumindest für einen bestimmten Zeitraum geschützt sind.

Alternativ zu der aufgezeigten, selbsttätig öffnenden und schließenden Klappe kann auch ein Rolladenelement vorgesehen werden, welches mit Hilfe entsprechend vorgesehener Stellmittel eine selbsttätige Abdeckung bzw. Freihaltung der an der Rückwand 15 vorgesehenen Öffnungen bzw. Anschlüsse bewirkt.

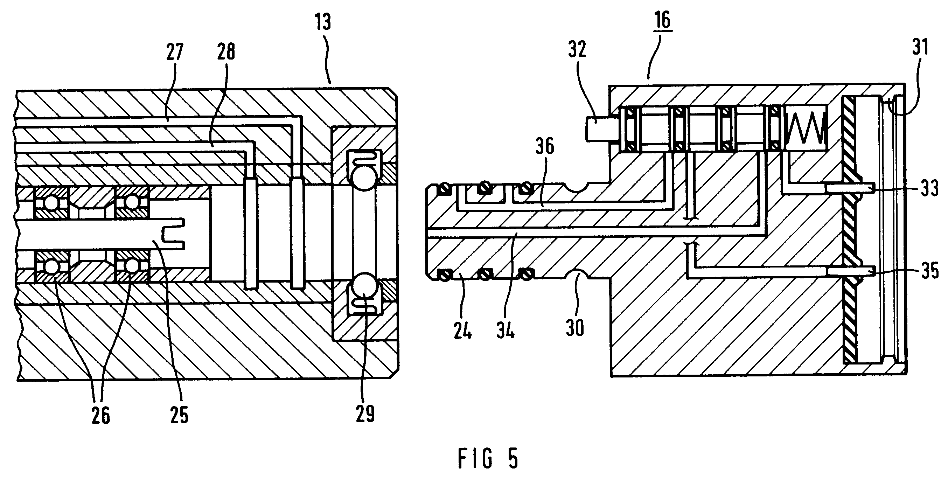

Anhand der Figur 5 wird am Beispiel des Adapters 16 der Aufbau und die Funktion der Adapter sowie das Zusammenwirken mit den daran aufsteckbaren Instrumenten erläutert.

Der Adapter 16 ist hier zum Anschließen eines in Figur 2 mit 13 bezeichneten Hand- und Winkelstückes vorgesehen, welches in seinem Inneren in Rotation versetzbare Triebwellen 25 sowie diesen zugeordnete Lager 26 enthält. Des weiteren verlaufen im Inneren eines solchen Handstückes Medienkanäle 27, 28 zur Zuführung von Kühlluft und Kühlwasser an die Präparationsstelle. Mit 29 ist eine Kugelrasteinrichtung bezeichnet, die beim Aufsetzen des Instruments 13 an einen diesem zugeordneten Antrieb mit einer entsprechenden Ringnut korrespondiert. Eine solche Ringnut ist beim Adapter 16 vorgesehen und dort mit 30 bezeichnet.

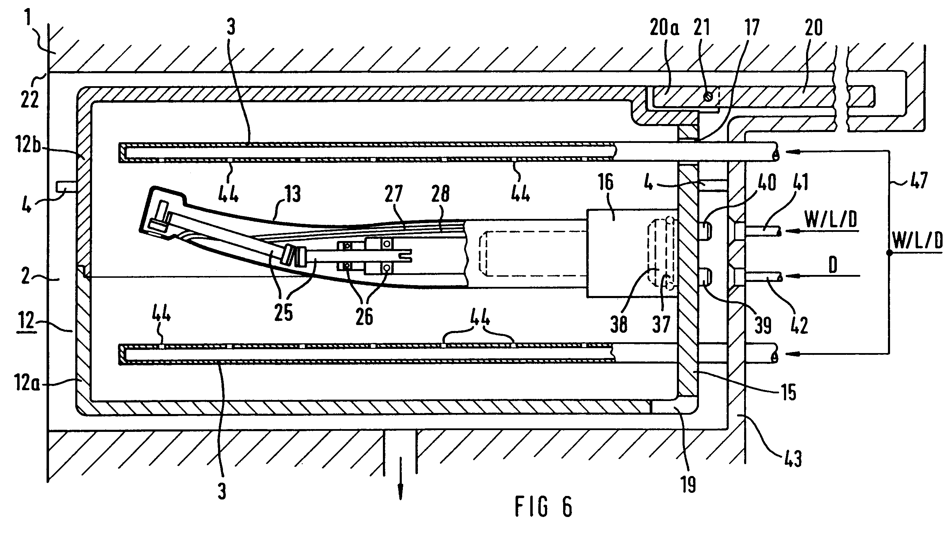

Der Adapter 16 enthält einen Anschlußzapfen 24, der baulich auf die Konstruktion des Instruments abgestimmt ist. An dem dem Instrument abgewandten Ende ist der Adapter 16 so ausgebildet, daß er an der Rückwand 15 des Kassettenunterteils 12a leicht lösbar befestigt werden kann. Hierzu enthält er eine Rastnase 31, die mit einer Ringnut 37 eines Flansches 38 (Fig. 6) an der Rückwand 15 des Kassettenunterteils 12a zusammenwirkt.

Im Adapter 16 befindet sich ein achsparallel verschiebbarer Schaltplunger 32, welcher beim Aufschieben des Instruments 13 entgegen der Kraft einer nicht näher bezeichneten Feder betätigt wird. Bei Betätigung wird der Eingangskanal 33 mit einem zentrisch gelegenen Kanal 34 verbunden. Außerdem wird die Verbindung eines weiteren Eingangskanales 35 mit einem Leitungskanal 36 hergestellt, der im aufgesetzten Zustand des Instruments über periphere Öffnungen mit den Medienleitungen 27, 28 des Instruments verbunden ist. Über den Eingangskanal 33 kann Treibluft oder Schmieröl zu den Triebwellen 25 und deren Lager 26 geleitet werden; über den Leitungskanal 35 kann Luft und Wasser gemeinsam zu den Leitungen 27 und 28 geführt werden.

Mit dem Plungerventil 32 ist sichergestellt, daß bei nicht mit einem Instrument belegtem Adapter die zugeführten Medien am Adapter nicht austreten können.

Wie bereits erwähnt, sind die Anschlußmaße bezüglich der Instrumente für alle vier, in der Kassette gehalterten Adapter unterschiedlich, um so unterschiedliche Instrumente haltern zu können. Die Anschlußmaße bezüglich des Anschlusses an der Rückwand 15 der Kassette sind jedoch für alle Adapter gleich, so daß sie sehr leicht untereinander ausgetauscht bzw. gegen solche mit anderen Anschlußkonturen für andere Instrumente gewechselt werden können.

Anhand der Figur 6 wird am Beispiel des Instruments 13 und des Adapters 16 die Zuordnung der einzelnen Wasch- und Reinigungsdüsen bzw. -kanäle erläutert.

Die Figur 6 zeigt in einer stark vereinfachten Darstellung die Anordnung der Kassette 12 in der Kammer 2 in nahezu vollständig eingeschobenem Zustand. An der Rückwand 15 der Kassette 12 befinden sich, entsprechend der Anzahl der vorhandenen Adapter, Anschlußflansche 38, auf die, wie bereits erwähnt, die Adapter 16 leicht lösbar aufgesetzt werden können. Die Anschlußflansche 38 weisen Verbindungsmuffen 39, 40 auf, welche bei völlig eingeschobener Kassette eine Verbindung herstellen, einerseits zwischen Zuleitungen 41, 42 und den bereits erwähnten Eingangskanälen 33 und 35 (Fig. 5). Die um das Instrument 13 stern- bzw. dreieckförmig herum angeordneten Düsenarme 3 sind an der Gehäusewandung 43, welche die Kammer 2 rückseitig begrenzt, fest angeordnet. Sie sind als Hohlleitungen ausgebildet und weisen eine Vielzahl von Düsenaustrittsöffnungen 44 auf, die unter einem bestimmten Winkel auf die Oberfläche des Instruments 13 ausgerichtet angeordnet sind. Die Düsenbohrungen sind vorteilhafterweise längs der Düsenarme nicht nur in einer Ebene, sondern in mehreren um einen bestimmten Winkel gegeneinander versetzten Ebenen angeordnet. Der Versatz kann vorteilhafterweise zick-zack- oder wellenförmig in Längsrichtung entlang einer Bezugsebene angeordnet sein.

Wie aus Figur 1 und auch aus dem nachfolgend noch näher erläuterten hydraulisch-pneumatischen Schaltplan hervorgeht, sind zur Aufbereitung von maximal vier Instrumenten vorteilhafterweise neun Düsenarme vorgesehen. Die Anordnung ist dabei so getroffen, daß die Düsenarme jeweils in einem Winkel von 120° zueinander um ein Instrument herum angeordnet sind.

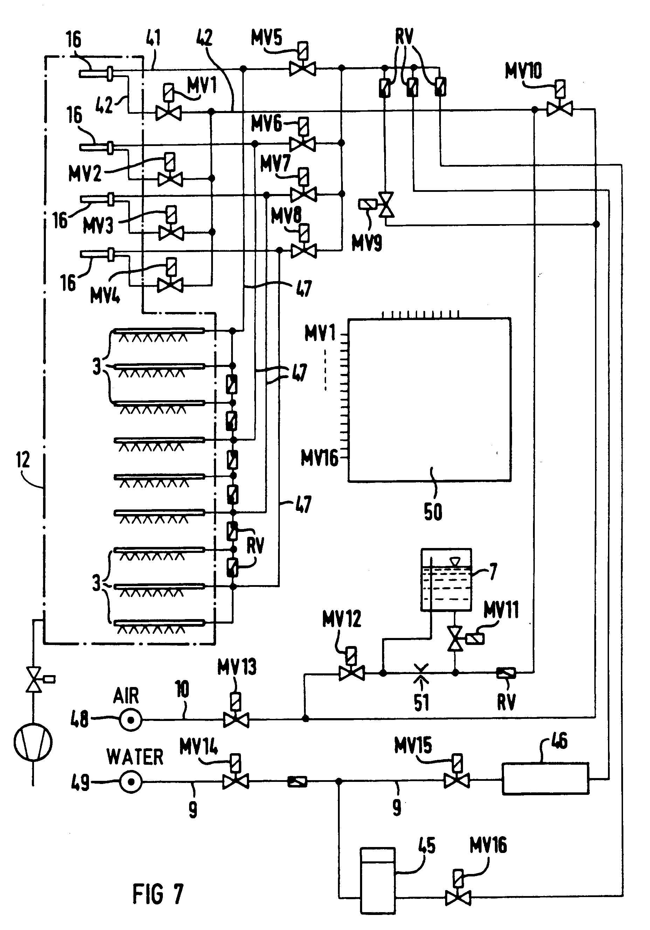

Bevor das erfindungsgemäße Verfahren anhand der bereits erläuterten Zeichnung und des hydraulisch-pneumatischen Blockschaltbildes gemäß Figur 7 näher erläutert wird, sei noch erwähnt, daß im Gerätegehäuse außer dem bereits erwähnten Ölbehälter 7 auch ein an die Druckwasserleitung 9 angeschlossener Dampferzeuger 45 sowie eine Vorwärmeinrichtung 46 für Wasser untergebracht sind.

Die Figur 7 zeigt ein hydraulisch-pneumatisches Schaltbild von der erfindungsgemäßen Einrichtung.

Mit 48 und 49 sind die Versorgungsquellen bezeichnet, mit denen einerseits Druckluft und andererseits Druckwasser in das Gerät eingespeist werden. Die eingangsseitig üblichen Filter, Ölabscheider (bei Druckluft) sowie Überdruckventile sind der Einfachheit halber nicht eingetragen.

Sämtliche Magnetventile (MV1 bis MV16) sowie die Wasserheizung 46 und der Dampferzeuger 45 werden von einem Mikroprozessor 50 aus gesteuert. Mit RV sind in die Leitungen geschaltete Rückschlagventile bezeichnet.

Die Injektion des Schmiermittels (mit Luft vermischbares Pflegeöl) aus dem Behälter 7 erfolgt hier pneumatisch über die beiden Magnetventile MV1 und MV 12 sowie eine Droselstelle 51. Alternativ kann das Schmieröl auch mittels einer geeigneten Ölpumpe zugeführt werden. Der MP 50 steuert die Magnetventile MV1 bis MV9 und MV11 bis MV16 so, daß den Adaptern 16 und den Düsenarmen 3 die Medien Luft, Wasser und gegebenenfalls das Schmieröl bei den Verfahrensschritten der Vor- und der Nachreinigung - und gegebenenfalls beim Pflegevorgang - für jedes Instrument getrennt zugeführt werden, so daß die Instrumente zeitlich nacheinander gereinigt - und gegebenenfalls gepflegt - werden.

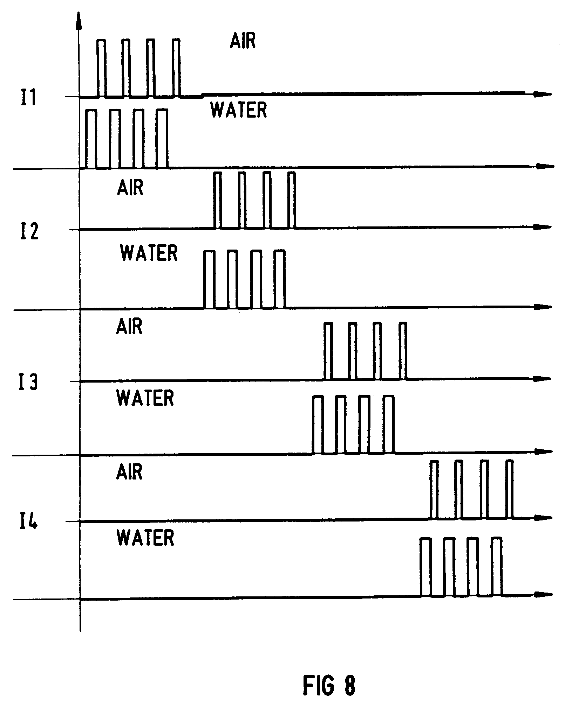

Aus Figur 8, die den zeitlichen Verlauf der Ventilanschaltung für die Medien Luft und Wasser für vier Instrumente (im Diagramm mit I1 bis I4 bezeichnet) aufzeigt, ist dieser zeitliche Versatz erkennbar. Mit dieser zeitlich aufeinanderfolgenden Zuschaltung der Medien wird bei niedrigem Wasserverbrauch eine hohe Reinigungseffizienz erzielt.

Das erfindungsgemäße Verfahren läuft wie folgt ab:

Zunächst wird die Kassette 12 mit Instrumenten beschickt. Instrumente, die bewegbare Innenteile haben, wie Winkelstücke, Turbinen, Spritzen, od.dgl., werden auf die entsprechenden Adapter 16 (max. vier) der Kassette aufgesteckt. Die übrigen Instrumente werden an Haltevorrichtungen über dem Behälterboden plaziert. Die Kassette wird sodann durch Aufsetzen des Kassettenoberteils verschlossen und in die Kammer 2 des Gerätes eingeführt. Mit dem Einführen wird die rückwärtige Abdeckung 20 angehoben, wodurch die neun Düsenarme 3 durch die Bohrungen 17 der Kassettenrückwand hindurchgreifen können. Nachdem die Kassette in der Kammer 2 so weit eingeschoben ist, daß die Verbindung der Leitungen 41, 42 mit den Anschlüssen 39, 40 (Fig. 6) hergestellt ist, wird der Deckel 5 das Gerät geschlossen und das Gerät eingeschaltet. Nach einem Selbsttest, der etwa eine halbe Minute dauert, erfolgt zunächst eine Kaltreinigung der Instrumente, indem über die Zuleitung 9 und die Leitungen 41 und 47 den Adaptern 16 und den Düsenarmen 3 kaltes Wasser zugeführt wird. Das kalte Wasser wird vorzugsweise pulsierend zugeführt, wobei dem Wasser in den Impulspausen Druckluft mit höherem Druck (ebenfalls pulsierend) beigemischt wird (Fig. 8). Dadurch wird ein hochenergetischer Wasserstrahl erzeugt, der überraschenderweise eine sehr gründliche Reinigung der Oberfläche und auch der Innenkanäle bewirkt. Gleichzeitig wird über die Leitung 42 der Getriebekanal mit Luft beaufschlagt (das Ölzuführungsventil MV11 bleibt geschlossen), wodurch das Eindringen von Schmutz und (verschmutztem) Reinigungswasser verhindert wird. Die Impulsfrequenz, mit der dem Wasser Druckluft zugeführt wird, beträgt etwa 3 Hz.

Die Kaltreinigung ist nach etwa 2 Minuten beendet. Danach erfolgt eine Warmreinigung der Außenflächen der Instrumente und auch der Innenkanäle, ebenfalls mit einem pulsierenden Wasserstrahl, unter Beifügung von gepulster Druckluft. Dieser Vorgang läuft wie vorerwähnt ab. Nach etwa 2 Minuten Warmreinigung erfolgt eine intensive Nachreinigung und Dampfdesinfektion durch Ab-und Ausblasen der Außenflächen der Instrumente und der Medien-und Getriebekanäle im Innern der Instrumente mit Heißwasser von 60° bis 100°C. Das Wasser wird hierzu von dem als Durchlauferhitzer wirkenden Erhitzer 46 erwärmt. Dieser Vorgang dauert etwa 30 bis 45 Sekunden.

Nach dieser intensiven Nachreinigung erfolgt eine Pflege der bewegten Innenteile durch Injektion einer dosierten Menge Schmieröl aus dem Schmierölvorratsbehälter 7. Die Zudosierung kann in Abhängigkeit vom adaptierten Instrument erfolgen und beträgt im Mittel 1 Gramm pro Instrument und Injektionszyklus.

Dieser Pflegeschritt dauert etwa eine halbe Minute. Anschließend erfolgt eine Sterilisation der Instrumente, und zwar sowohl von außen als auch von innen, mittels gesättigtem Wasserdampf von vorzugsweise 134°C bei einem Druck von etwa 2 bis 2,5 bar. Der Sterilisationsvorgang beträgt zwischen 5 und 6 Minuten. Danach folgt ein Trocknen und Abkühlen der Instrumente mittels kalter Luft. Der Abkühlvorgang dauert etwa 3 bis 5 Minuten. Bei einer Gesamtzeit von etwa 15 bis 20 Minuten zur Aufbereitung der Instrumente kann danach die Kassette mit den aufbereiteten Instrumenten entnommen und im geschlossenen Zustand zur Benutzung bereitgestellt werden.

Der Verfahrensablauf, der an sich selbsttätig in der geschilderten Weise abläuft, kann durch Eingriff, z.B. durch entsprechende Programmvorwahl, die an einem Bedien- und Displayfeld 52 an der Frontseite des Gerätes abgerufen werden kann, variiert werden, z.B. indem im Anschluß an die intensive Nachreinigung und Desinfektion der Außenfläche eine Zwischentrocknung, z.B. durch Luft, durchgeführt wird. Ebenso kann nach dem Pflegen eine Unterbrechung stattfinden, um so nicht sterilisierbare Gegenstände aus der Kassette entnehmen zu können.

Zur Entfernung vorhandener Trockenluft, insbesondere aus Hohlräumen, und im besonderen aus Hohlräumen der Instrumente, kann gemäß einer vorteilhaften Variante die Kammer vor dem Sterilisieren der Instrumente evakuiert werden. Um eine optimale Entfernung der Trockenluft zu erzielen, sollte das Vakuum im Bereich zwischen 40 und 500 mbar absolut liegen. Das Vakuum kann nach dem an sich bekannten fraktionierten (mehrstufigen) Verfahren oder nach dem Vorvakuumverfahren erzeugt werden.

Mit dem geschilderten Verfahren ergeben sich extrem kurze Zeiten für eine optimale hygienische Aufbereitung der Instrumente. Durch den pulsierenden Wasserstrahl, dem stoßweise Druckluft höherer Energie zugeführt wird, ist eine besonders intensive Reinigung der Teile gewährleistet. Dadurch, daß nach der Aufbereitung die Instrumente in der Kassette verbleiben können, ist ein sicherer Transport zum Behandlungsplatz gegeben. Das gleiche gilt für den Weg nach Benutzung der Instrumente zur Aufbereitung. Die nach der Benutzung mit Keimen kontaminierten Instrumente verbleiben bis zur Wiederentnahme in der Kassette, wodurch u.a. eine Verletzungsgefahr für das Bedienpersonal ausgeschlossen ist. Bearbeitung, Lagerung und Transport erfolgen somit in einer Kassette. Nachdem die Kassette weitgehend abgeschlossen ist, ist eine sterile Lagerung der Instrumente zumindest über einen hinreichend langen Zeitraum (Stunden) gewährleistet. Dadurch, daß die Innenteile und die Medienkanäle mit Heißwasser und Dampf durchströmt werden, ergibt sich eine relativ rasche Aufheizzeit der gesamten Instrumente. Durch das pulsierende Ab- und Ausblasen der Teile mit Wasserdampf wird auch ein gewisser kalklösender Effekt in den Leitungen erzielt. Ein wesentlicher Vorteil ist, daß keinerlei Reinigungs- und Treibmittel verwendet zu werden brauchen.

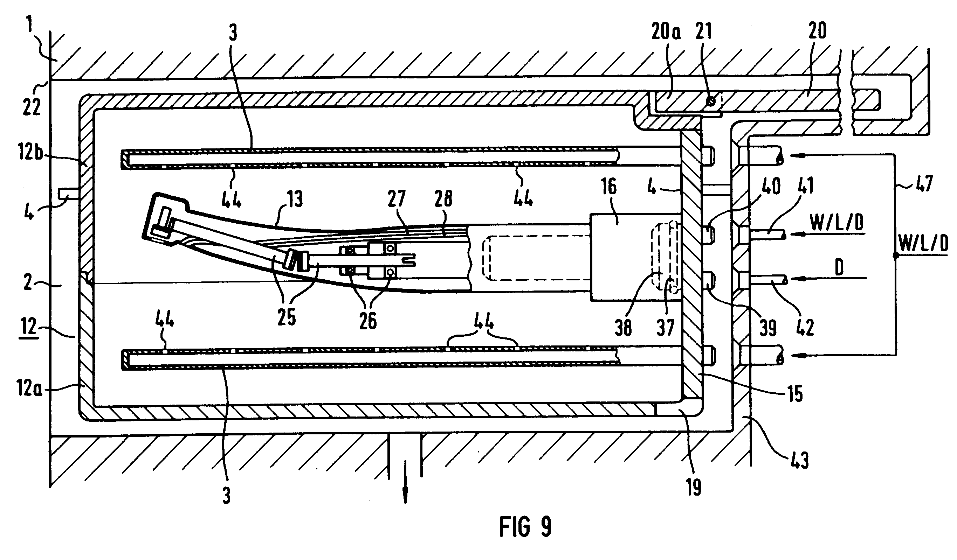

Hinsichtlich der Anordnung und Ausbildung der Düsenarme 3 sind im Rahmen der Erfindung verschiedene Modifikationen denkbar. So können beispielsweise die Düsenarme 3 auch Bestandteil der Kassette sein, wie dies im Ausführungsbeispiel nach Fig. 9 dargestellt ist. Des weiteren könnten, obgleich dies relativ aufwendig wäre, die Düsenarme um ihre Längsachse schwenkbar und gegebenenfalls auch in Achsrichtung bewegbar angeordnet sein. Die in den Fig. 3 bis 5 dargestellte Abdeckung kann alternativ auch Bestandteil des Kassettenunterteils sein oder beiden Kassettenhälften zugeordnet sein.

## Patentansprüche

1. Kassette zur Aufnahme von ärztlichen, insbesondere zahnärztlichen Instrumenten, die zum Zwecke einer hygienischen Behandlung in einer druckdicht verschließbaren Kammer eines Gerätes einlegbar sind und dort einem Behandlungsprozeß unterworfen werden, wobei die Kassette (12) mindestens einen Adapter (16) zur Halterung mindestens eines Instruments (13) aufweist,
**dadurch gekennzeichnet,**
daß mindestens ein Adapter (16) Leitungskanäle (33 bis 36) zur Zuführung der Behandlungsmedien in das Innere der Instrumente (13) aufweist und ein Steuerventil (32) beinhaltet, welches die Leitungskanäle (34, 36) ) bei nicht mit einem Instrument (13) belegtem Adapter (16) verschließt.

2. Kassette nach Anspruch 1,
**dadurch gekennzeichnet,**
daß mehrere Adapter (16) für unterschiedliche Instrumente (13) vorgesehen sind, wobei die Adapter bezüglich ihrer Halterung an einer Gehäusewand (15) der Kassette gleiche Anschlußmaße aufweisen.

3. Kassette nach Anspruch 2,
**dadurch gekennzeichnet,**
daß die Adapter (16) leicht auswechselbar an der Rückwand (15) der Kassette (12) gehaltert sind.

4. Kassette nach einem der Ansprüche 1 bis 3,
**dadurch gekennzeichnet,**
daß die Kassette (12) aus zwei durch Querteilung gebildeten und miteinander verbindbaren Gehäusehälften (12a, 12b) besteht, wobei die bodenseitige Gehäusehälfte (12a) den/die Adapter (16) trägt und die deckseitige Gehäusehälfte (12b) Führungselemente (4) zur Führung der Kassette (12) in der Kammer (2) des Gerätes aufweist.

5. Kassette nach Anspruch 3,
**dadurch gekennzeichnet,**
daß die Rückwand (15) mit Öffnungen (17) versehen ist, durch die beim Einschieben der Kassette in die Kammer (2) dort befestigte Düsenarme (3) hindurch-greifen können, wobei die Öffnungen in der Kassette für den Durchtritt der Düsenarme von einem Abdeckmittel (20) verschlossen werden und dem Abdeckmittel ein Steuermittel (20a) zur Freigabe der Öffnungen zugeordnet ist.

6. Kassette nach Anspruch 5,
**dadurch gekennzeichnet,**
daß das Abdeckmittel eine an der Rückwand (15) der Kassette (12) schwenkbar gehalterte Platte (20) ist, die einen Überstand (20a) aufweist, der beim Einführen der Kassette in die Kammer (2) an einer Gehäusekante (22) zu Anlage kommt und dabei die Platte im Sinne einer Freigabe der Öffnungen (17) verstellt.

7. Kassette nach einem der vorangegangenen Ansprüche,
**dadurch gekennzeichnet,**
daß sie aus einem sterilisierbaren Kunststoff besteht.

8. Kassette nach einem der Ansprüche 1 bis 4,
**dadurch gekennzeichnet,** daß an ihr ein oder mehrere Düsenarme (3) befestigt sind, deren Anschlußstutzen beim Einsetzen in die Kammer mit den Zuleitungen (47) der Behandlungsmedien in Eingriff kommen.

## Claims

1. Cassette for receiving medical, in particular dental instruments, which for the purpose of obtaining hygienic treatment can be placed in a chamber of a device that cab be sealed in a pressure-tight manner and are there subjected to a treatment process, with the cassette (12) having at least one adaptor (16) for holding at least one instrument (13), characterized in that at least one adaptor (16) has line channels (33 to 36) for the purpose of feeding the treatment agents into the interior of the instruments (13) and contains a control valve (32) which seals the line channels (34, 36) when the adaptor (16) is not occupied by an instrument (13).

2. Cassette according to claim 1, characterised in that a plurality of adaptors (16) are provided for different instruments (13), wherein the adaptors have the same connection dimensions with respect to their holding support on a housing wall (15) of the cassette.

3. Cassette according to claim 2, characterised in that the adaptors (16) are held on the rear wall (15) of the cassette (12) in such a way that they can easily be exchanged.

4. Cassette according to one of the claims 1 to 3, characterised in that the cassette (12) consists of two housing halves (12a, 12b) which are formed by transverse division and which can be connected together, wherein the housing half (12a) on the base side bears the adaptor/adaptors (16) and the housing half (12b) on the top side has guide elements (4) for guiding the cassette (12) in the chamber (2) of the device.

5. Cassette according to claim 3, characterised in that the rear wall (15) is provided with openings (17) through which, upon insertion of the cassette into the chamber (2), nozzle arms (3) which are secured there can be engaged, wherein the openings in the cassette for the through-passage of the nozzle arms are sealed by a cover means (20), and a control means (20a) for freeing the openings is associated with the cover means.

6. Cassette according to claim 5, characterised in that the cover means is a plate (20) which is held on the rear wall (15) of the cassette (12) in a pivotable manner and has a projection (20a) which, upon introduction of the cassette into the chamber (2), comes to rest against a housing edge (22) and thereby displaces the plate for the purpose of freeing the openings (17).

7. Cassette according to one of the preceding claims, characterised in that it is made of a plastics material which can be sterilized.

8. Cassette according to one of the claims 1 to 4, characterised in that one or a plurality of nozzle arms (3) is/are secured thereon, the connecting branches of which arms, when inserted into the chamber, are engaged with the feed lines (47) pertaining to the treatment agents.

## Revendications

1. Cassette destinée à recevoir des instruments médicaux, notamment des instruments de dentisterie, qui, dans le but d'un traitement hygiénique, doivent être placés dans une chambre, pouvant être fermée de façon étanche à la pression, d'un appareil et y être soumis à un processus de traitement, la cassette (12) comportant au moins un adaptateur (16) destiné à retenir au moins un instrument (13),
caractérisée par le fait que,
au moins un adaptateur (16) comporte des canaux de passage (33 à 36) destinés à l'amenée des fluides de traitement à l'intérieur des instruments (13), et comporte une vanne de commande (32) qui obture les canaux de passage (34, 36) lorsque l'adaptateur (16) n'est pas occupé par un instrument (13).

2. Cassette selon la revendication 1,
Caractérisée par le fait que
plusieurs adaptateurs (16) sont prévus pour des instruments (13) différents, les adaptateurs ayant les mêmes cotes de raccordement en ce qui concerne leur fixation sur une paroi (15) du boîtier de la cassette.

3. Cassette selon la revendication 2,
caractérisée par le fait que
les adaptateurs (16) sont fixés sur la paroi arrière (15) de la cassette (12) de façon à être facilement interchangeables.

4. Cassette selon I'une quelconque des revendications 1 à 3,
caractérisée par le fait que
la cassette (12) est constituée de deux moitiés de boîtier (12a, 12b) formées par séparation transversale et pouvant être reliées entre elles, la moitié de boîtier (12a) située du côté du fond portant le/les adaptateur(s) (16), et la moitié de boîtier (12b) située du côté du couvercle comportant des éléments de guidage (4) destinés au guidage de la cassette (12) dans la chambre (2) de l'appareil.

5. Cassette selon la revendication 3,
caractérisée par le fait que
la paroi arrière (15) est munie d'orifices (17) par lesquels peuvent passer, lorsque la cassette est insérée dans la chambre (2), des bras de buse (3) qui y sont fixés, les orifices pratiqués dans la cassette pour le passage des bras de buse étant obturés par un élément de recouvrement (20), et un élément de commande (20a) étant associé à l'élément de recouvrement pour dégager les orifices.

6. Cassette selon la revendication 5,
caractérisée par le fait que
l'élément de recouvrement est une plaque (20) retenue de façon à pouvoir basculer sur la paroi arrière (15) de la cassette (12) et comportant une partie en saillie (20a) qui est appliquée, lorsque la cassette est insérée dans la chambre (2), contre le bord supérieur (22) du boîtier et qui déplace la plaque en dégageant les orifices (17).

7. Cassette selon l'une quelconque des revendications précédentes,
caractérisée par le fait que
elle est constituée d'une matière plastique pouvant être stérilisée.

8. Cassette selon l'une quelconque des revendications 1 à 4,
caractérisée par le fait que
il y est fixé un ou plusieurs bras de buse (3), dont les tubulures de raccordement sont mises en liaison avec les lignes d'alimentation (47) des fluides de traitement lors de l'insertion dans la chambre.
